# EUROPEAN PATENT APPLICATION

(11) **EP 3 937 105 A1**
(43) Date of publication of application: **12.01.2022**
(21) Application number: 20184233.3
(22) Date of filing: 06.07.2020
(51) Int. Cl.: G06Q 10/10, G06Q 50/26, G16H 10/00, G16H 10/20

(54) **METHODS AND SYSTEMS FOR USER DATA PROCESSING**

(71) Applicant: Koninklijke Philips N.V., 5656 AG Eindhoven (NL)
(72) Inventor: LI, Zuofeng, 5656 AE Eindhoven (NL); WEN, Dong, 5656 AE Eindhoven (NL); TAO, Liang, 5656 AE Eindhoven (NL); CHOU, Hsu-Wen, 5656 AE Eindhoven (NL); HU, Yiyi, 5656 AE Eindhoven (NL); ZHOU, Cloudy, 5656 AE Eindhoven (NL); HU, Eva, 5656 AE Eindhoven (NL)
(74) Representative: Philips Intellectual Property & Standards

(57) **Abstract**

The invention provides a method for automatically filling a digital report form with relevant user data. The method includes obtaining a digital report form and extracting an input data model from the digital report form. A query is then generated based on the input data model. A digital user record is obtained, relevant user data is identified in the digital user record based on the query and extracted. The digital report form is then filled based on the relevant user data.

## Description

### FIELD OF THE INVENTION

The invention related to the field of processing user data, and more specifically to the field of user data extraction based on automatically generated search criteria.

### BACKGROUND OF THE INVENTION

Clinical research has long depended on manual data collection instruments, such as case report forms (CRFs), to structure and facilitate collection of data for clinical trials. Most CRFs are customized to collect data specific to a particular clinical study protocol.

Historically, CRFs were paper-based (pCRF); however, there has recently been a shift towards the use of electronic CRFs (eCRFs). eCRFs have led to an increase in data quality and completeness by using error alarms, automatic data completion and reminders for data entry required at a later date.

Typically, the benefits of electronic data capture (EDC) outweigh the challenges; however, it requires continual reassessment and re-evaluation of novel processes as they are developed and implemented. Therefore, while the use EDC has steadily increased, paper is still used when EDC is unfeasible for logistic or financial reasons.

Some EDC systems may extract or copy data from medical records, or other system data, for entry into the eCRF; however, this requires input from IT engineers or clinical domain experts to perform data mapping in order obtain the relevant data. Data mapping is a resource-intensive project requiring hands-on review and considerable knowledge about the source data and target data.

Therefore, EDC is generally employed as part of large projects with sufficient resources in order to implement it. However, projects with limited resources are generally required to resort to manually data entry, leading to a reduction in the data quality of these projects.

There is therefore a need improve the accessibility and quality of automated EDC.

Further, there has been an increase in clinical trials conducted globally and registered in public international databases. Each registered clinical trial has eligibility criteria information, which describes the demographic and medical characteristics that a research volunteer must possess in order to participate in the clinical trial. Generally, the criteria are divided into two sections: inclusion criteria and exclusion criteria, which are typically held in unstructured free text.

Currently, eligibility criteria are available only in free text, which is difficult to parse or process computationally. Therefore, eligibility screening is still conducted manually, which typically requires a lengthy review of patient records and is a labor intensive process.

More specifically, physicians are required to review whether a patient is qualified for a clinical trial and then inform a research team to take over the screening activities. The researchers may then develop algorithms using the data in the patient health record to detect the patient phenotype. Typically, the phenotyping process extracts features from the patients' medical record and assembles them into a phenotyping algorithm to infer whether the patient has a target phenotype. The process is typically tedious and long and generally requires IT engineering to write a specific query code.

There is therefore also a need to provide an improved means of automatically assessing the eligibility of a user for a clinical trial.

### SUMMARY OF THE INVENTION

The invention is defined by the claims.

According to examples in accordance with an aspect of the invention, there is provided a method for automatically filling a digital report form with relevant user data, the method comprising:
obtaining a digital report form;
extracting an input data model from the digital report form;
generating a query based on the input data model;
obtaining a digital user record;
identifying relevant user data in the digital user record based on the query;
extracting the relevant user data; and
filling the digital report form based on the relevant user data.

The method provides a means of automatically extracting relevant user data from a digital user record for filling in a digital form.

By extracting only the relevant user data for filling in the form based on an input data model extracted from said form, the accuracy of the form completion may be increased.

In an embodiment, the method further comprises obtaining context information from the digital report form, and wherein the generating of a query is further based on the context information.

In this way, with the analysis of more context information, the query for targeting the answer of the data element is more accurate, and the relevant user data may be more accurately identified.

In a further embodiment, the context information comprises multiple entities.

In a further embodiment, generating the query comprises:
grouping multiple entities of the context information from at least the digital report form;
generating multiple query sequences based on entities in at least one group; and
deriving the query of the input data model by comparing the multiple query sequences with the input data model.

In this way, the generated query may more accurately match the context of the digital report form and the relevant user data.

In an embodiment, the input data model comprises:
a timestamp;
a user identifier; and
a data element.

In this way, the input data model may encompass a variety of input data.

In a further embodiment, the data element comprises one or more of
a semantic definition;
a document type;
a numerical value;
a numerical range; and
a unit.

In this way, the input data model, and specifically the data element, may include a wide variety of input data, thereby accommodating for a wider range of applications.

In a further embodiment, the semantic definition comprises one or more of:
a conditional statement;
a confirmation; and
a negation.

In this way, the input data model may take the language of the digital form into account.

In an embodiment, the input data model comprises a data element having a data element type, and wherein the data element type comprises:
a finite choice; or
a free text entry.

In this way, the query can be used to search the digital user record for the correct type of relevant user data.

In an embodiment, extracting the input data model comprises extracting the data element, and wherein extracting the data element comprises:
determining if the data element comprises:
   a check box;
   a table cell; or
   a string entry field;
if the data element comprises a check box, determining if the check box comprises a predefined option;
   if the check box comprises a predefined option, identifying the data element as a finite choice;
   if the check box does not comprise a predefined option, identifying the data element as a free text entry;
if the data element comprises a table cell, identifying the data element as a free text entry; and
if the data element comprises a string entry field, identifying the data element as a free text entry.

In an embodiment, the method further comprises:
identifying a data element of the input data model as a fake data element;
discarding the fake data element; and
obtaining a new data element from the digital report form.

In this way, errors may be accounted for, thereby increasing the accuracy of the form completion.

In a further embodiment, the obtaining of the context information comprises applying a top-down algorithm, the top-down algorithm comprising:
identifying a page of the digital report form;
identifying a heading on the page; and
deriving the context information based on the heading.

In an embodiment, the obtaining of the context information comprises applying a bottom-up algorithm, the bottom-up algorithm comprising:
applying a leaf entity matching algorithm to an entity of the digital report form;
identifying a similar entity based on the leaf matching of the entity; and
deriving the context information based on the similar entity.

In an embodiment, the method further comprises generating a data alert for displaying to a user.

In this way, the user may be informed of matters that may require their attention.

In an embodiment, the data alert comprises an error flag.

In an embodiment, the method further comprises, if no relevant user data can be extracted, receiving a user input to provide relevant user data.

In this way, a user may account for missing information.

According to examples in accordance with an aspect of the invention, there is provided a computer program comprising computer program code means which is adapted, when said computer program is run on a computer, to implement the methods described above.

According to examples in accordance with an aspect of the invention, there is provided a system for automatically filling a digital report form with relevant user data, the system comprising a processor adapted to:
obtain a digital report form;
extract an input data model from the digital report form;
generate a query based on the input data model;
obtain a digital user record;
identify relevant user data in the digital user record based on the query;
extract the relevant user data; and
fill the digital report form based on the relevant user data.

According to examples in accordance with an aspect of the invention, the method further comprises identifying an eligible user using a text based criterion and obtaining the digital user record is further based on the identified eligible user, wherein the method of identifying the eligible user comprises:
obtaining text data, wherein the text data comprises the text based criterion;
decomposing the text based criterion into one or more sub-sentences;
decomposing the one or more sub-sentences into one or more semantic phrases;
identifying each semantic phrase as a search feature;
generating a search criterion based on the one or more search features;
searching a user database based on the search criterion; and
identifying an eligible user based on the search of the user database.

The method provides an automated means for extracting a search criterion from a text document for use in searching a patient database for eligible patients.

The decomposing of the text data into sub-sentences and semantic phrases allows for the simplification of the text data into searchable elements.

In an embodiment, the text based criterion comprises a temporal element and wherein the search feature comprises a temporal criterion.

In this way, a time component may be included in the search criterion, which is typically important for clinical situations (such as clinical trials).

In an embodiment, the method further comprises assigning the sub-sentences to a group, wherein the group comprises:
a general group; and
a first order difference group, wherein the first order difference group is dependent on the general group.

In this way, it is possible to establish a hierarchy within the criterion relating to the importance of a given criterion element.

In a further embodiment, the assigning of the sub-sentences to a group is based on a temporal element.

In an embodiment, the method further comprises:
comparing the search feature to a medical database; and
updating the search criterion based on the comparison.

In this way, a database, such as a Fast Healthcare Interoperability Resources (FHIR) database, may be referenced using the entities.

In an embodiment, the search feature comprises an entity of the text based criterion, wherein the entity comprises one or more of:
a medicament identity;
a medical condition;
a laboratory; and
a medical examination.

In an embodiment, the search feature comprises a feature of the text based criterion, wherein the feature of the text based criterion comprises one or more of:
an arithmetic comparator;
an affirmation;
a negation; and
a conditional statement.

In an embodiment, the search feature comprises a value of the text based criterion, wherein the value of the text based criterion comprises one or more of:
a numerical value;
a numerical range; and
a unit.

In an embodiment, the method further comprises:
providing the one or more sub-sentences to a user;
receiving a user input on the one or more sub-sentences; and
updating the sub-sentences based on the user input.

In this way, a user, such as a clinician, may see the sub-sentences and alter them. This may be used to train an automated system.

In an embodiment, the method further comprises generating a logic tree based on the search criterion.

In an embodiment, the text data comprises structured data and unstructured data.

In other words, the method may handle any input text data.

In an embodiment, the obtaining of the text data comprises one or more of:
natural language processing;
machine learning; and
information extraction.

According to examples in accordance with an aspect of the invention, there is provided a computer program comprising computer program code means which is adapted, when said computer program is run on a computer, to implement the methods described above.

According to examples in accordance with an aspect of the invention, there is provided a processing unit adapted to:
obtain text data, wherein the text data comprises a text based criterion;
decompose the text based criterion into one or more sub-sentences;
decompose the one or more sub-sentences into one or more semantic phrases;
identify each semantic phrase as a search feature;
generate a search criterion based on the one or more search features;
search a user database based on the search criterion; and
identify an eligible user based on the search of the user database.

According to examples in accordance with an aspect of the invention, there is provided a data processing system, the system comprising:
a processing unit as described above; and
a user interface in communication with the processing unit and adapted to receive a user input.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a method according to an aspect of the invention;
Figure 2 shows a schematic representation of generating a query based on a digital report form; and
Figure 3 shows a method according to a further aspect of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for automatically filling a digital report form with relevant user data. The method includes obtaining a digital report form and extracting an input data model from the digital report form. A query is then generated based on the input data model. A digital user record is obtained, relevant user data is identified in the digital user record based on the query and extracted. The digital report form is then filled based on the relevant user data.

A further aspect of the invention provides a method for identifying an eligible user for a clinical trial using a text based criterion. The method includes obtaining text data, wherein the text data comprises the text based criterion, decomposing the text based criterion into one or more sub-sentences and decomposing the one or more sub-sentences into one or more semantic phrases. Each semantic phrase is then identified as a search feature and a search criterion is generated based on the one or more search features. A user database is searched based on the search criterion and an eligible user identified.

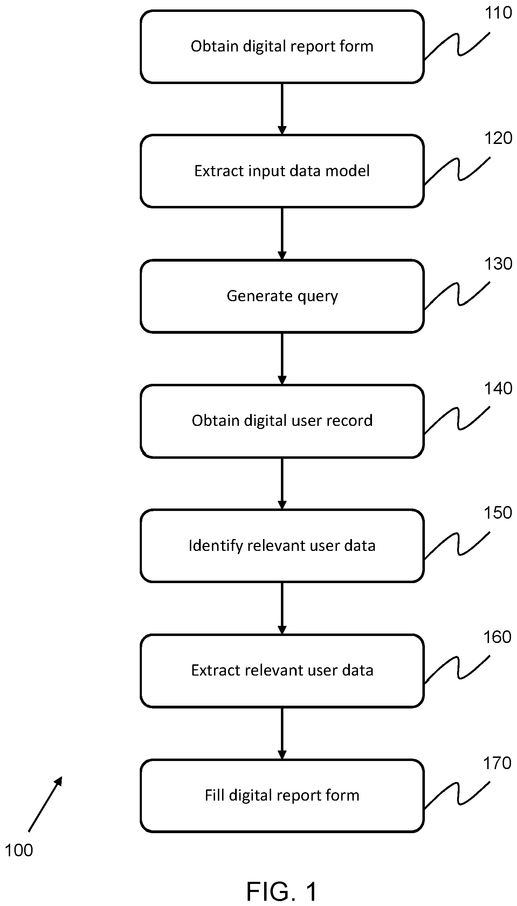

Figure 1 shows a method 100 for automatically filling a digital report form with relevant user data.

The method begins in step 110 by obtaining a digital report form.

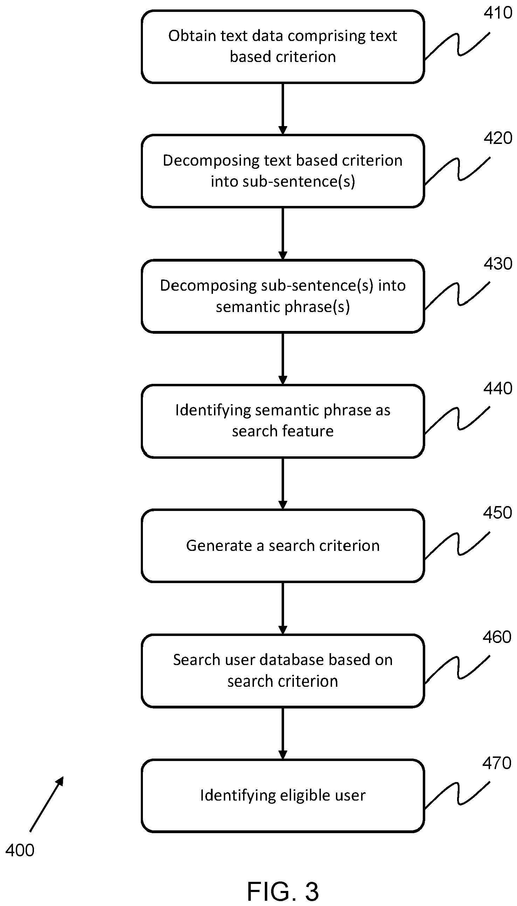

A digital report form may be any digital form for receiving data relating to a user. A digital report form may include one or more inclusion or exclusion criteria, which are discussed in more detail further below with reference to Figure 3.

In step 120, an input data model is extracted from the digital report form. The input data model relates to the data to be received by the digital report form. The input data model may include a data element, which may comprise one or more of: a semantic definition, such as a conditional statement, a confirmation and a negation; a document type; a numerical value; a numerical range; and a unit. The semantic definition may determine the type or the value of data extracted from database and filled into the data element.

Further, the input data model may include context information relevant to the data element. For example, the context information of the input data model may include multiple entities. An entity is any type of event, such as a body examination or diagnosis. The context information of the input data model may include a timestamp, which may relate to a time relevant to the data entered into the form, such as, the time that a medical event occurred. The context information of the input data model may also include a user identifier, which may then be used to retrieve the data relating to the user in question.

In use, each data element may be assigned an ID, which is determined based on the context information that appears in the digital report form.

In step 130, a query is generated based on the input data model. In other words, the input data model is used to generate a question to be answered using data relating to the user.

More specifically, each data element of the input data model may correspond to a query. The query may be generated based on entity information of the data element. In other words, the query may include entity information that is relevant to context information of each data element.

The data elements of the data input model may be grouped into a plurality of groups according to their type. Three exemplary groups may include: a mutual exclusion group; a split group; and an independent group. The data elements of the data input model may be related to each other in different ways. For example, two data elements may be mutually exclusive, meaning that only one should be selected. Further, some data elements may be split into several data elements, such as a date of birth that may be split into day, month and year. Further, some data elements are independent and have no relation to other data elements of the data input model.

Each of the mutual exclusion group and the split group may be assumed to have the same semantic meanings for the context information or the entities in the same group, meaning context information may be assigned at data element group level. In some embodiments, the data elements of the independent group may also share the same context information. In some embodiments, the nearest information to the data element of a given group may be used as context information. In some embodiments, simply selecting the nearest information as the context information may not be sufficient.

Accordingly, it may be necessary to establish the boundary of the context information for a given data element, wherein the boundary dictates the relevance of information in the vicinity of the data element. There are two approaches that may be utilized to establish the boundary of the context information.

In the first approach, referred to as a top-down approach, fonts, section names and headings are used to detect the pages, sections and sub-headings of the digital report form, respectively. The data element or grouped data elements, with the nearest context information will be assigned to one, or multiple, of the pages, sections and sub-headings. For example, data elements sharing the same page may be associated with the same context information.

In the second approach, referred to as a bottom-up approach, a leaf entity matching is applied, looking for the same or similar definition entity for each entity of the digital report form. A similar entity based on the leaf matching of the entity is identified. Finally, the context information based on the similar entity is obtained. In some embodiments, the bottom-up approach is implemented on the basis of the top-down approach.

A phenotyping algorithm, which is the algorithm used to generate the query and extract the relevant user data from the user data record based on the generated query, is implemented.

More specifically, based on the context information extracted by implementing the first and second approaches, or based on the context information extracted from any types of user data record, such as EMR record, imaging records, diagnosis results, patients notes, and the like, multiple driver events or entities are extracted from the context information first and then grouped. The entity extraction and grouping steps may be performed during the query generating process, or may be performed before the query generating process. The extracted entity information and the grouping information may be stored in a database.

As an example, ontology may be used to define a group of entities with same semantic meanings, such as Carcinoembryonic antigen and CEA. More specifically, driver events for the treatment of oncology, such as the term entities: Transhepatic Arterial Chem Otherapy And Embolization (TACE), liver transplantation, hepatectomy, and alinjection are grouped as one group at the first level. Further, the term entity alinjection in the first level may happen at the first operation, at the second operation, at the third operation. When designing the digital report form, the clinical researcher may hope to identify the subject that received alinjection at either the first operation, the second operation, the third operation or any one of those operations. Therefore, the term entities: at the first operation, at the second operation, at the third operation and at any one of those operations are grouped as one group at the second level. Similarly, different term entities can be grouped at different group levels, such as third level, fourth level, fifth level, and etc.

The context information in one group of entities may be similar at the same level. Each group may include multiple entities. Different groups of entities may be linked together according to different criteria. Each entity in a group may be combined with an entity from another group, which may be manually selected by the user or automatically generated by the system. The path is the combination of different entities in across the different groups.

After grouping the entities, multiple query sequences are generated based on entities in at least one group, each query sequence being semantically unique. More specifically, multiple query sequences may be generated since one data element may be relevant to different entities in different groups at different levels. However, only one sequence is the target query based on the context information of the data element. The target query is determined by the data element, and more specifically, by comparing the context information of the data element of one digital report form with the multiple query sequences. The closest matched result is then selected as the target query.

In an exemplary embodiment, leaf entity matching is then applied, looking for the same or similar definition entity for each data element. A leaf entity is the entity of the group at the last level. For example, where entities are grouped in 8 group levels, then the leaf entity is an entity at the 8th level.

However, the mapping may not be a simple one-to-one mapping, for example, because the expression of the entity in the algorithm may be different from the expression of the entity used in the digital report form. For example, in the digital report form, both the full name Carcinoembryonic antigen and the abbreviation CEA could be used.

In this case a bottom-up approach may be adapted, starting with the detected data element, which acts as a leaf entity, and tracing back all of the possible sequences to the root. In each sequence, multiple entities may be used for definition. Further, each entity in the definition sequence may be extended with synonyms. Moreover, each entity may be extended for multiple languages.

The extended entities may be used to match the entities detected in the digital report form. Finally, the matched entities will combine into one or multiple paths defining a phenotyping algorithm. The phenotyping algorithm with the maximum number of matched entities will be selected. The phenotyping algorithms with the same maximum number off matches may also be returned. Therefore, the definition of the phenotyping algorithms may be dynamic and change according to the data elements present in the digital report form. The phenotyping algorithms with the maximum number of matched entities will be assigned as the final phenotyping algorithm and the relevant user data will be retrieved with this final phenotyping algorithm.

The query may further include the type or the value of the data element. As an example, the type or the value of the data element may include a finite choice, such as a yes/no answer or a list of answers to select from. Alternatively, the type or the value of the data element may include a free text entry.

For example, the query may be generated by determining if the data element comprises a check box, or any other type of binary selection, and if the data element does comprise a check box, determining if the check box comprises a predefined option, such as whether the user has a family history of a given condition. If the check box comprises a predefined option, the data element may be defined as a finite choice; whereas, if the check box does not comprise a predefined option, the data element may be defined as a free text entry.

In a further example, if it is determined that the data element comprises a table cell, the data element may be defined as a free text entry. In another example, if it is determined that the data element comprises a string entry field, the data element may be identified as a free text entry.

Handling potentially erroneous data may be performed in a variety of ways. For example, an error flag may be presented where potentially erroneous data is detected and the user may be prompted to check the data. The potentially erroneous data may be detected by comparing the corpus difference between training and testing datasets of the natural language processing (NLP) methods employed to give an overall estimate the performance of NLP for data extraction. In addition, domain knowledge relating to the medical field of the digital report form may be used to define clinical logic tests to screen the extracted data for conflicts or inconsistencies.

For the data elements acquired by machine learning (ML) algorithms, the system performance may be improved by recording user decisions in response to error flags. The corrected data may be used to refill the training dataset and the model may be retrained accordingly. Further, the system may be adapted to recognize semantic confusion based on context information and prompt the user to clearly define the value in question.

In step 140, a digital user record is obtained and in step 150, relevant user data is identified in the digital user record based on the query. The digital user record may be obtained from any available source of data relating to the user. The available source may include EMR, HIS, RIS, PACS, patients' health notes and the like.

In step 160, the relevant user data is extracted from the digital user record and in step 170, the digital report form is filled based on the relevant user data.

The relevant data may be extracted from the digital user record according to the following methods.

For each data source, the minimum information may be extracted first. In the input data model, two obligatory data elements may include a timestamp relating to a medical event and a patient identification. The timestamp may include a sequence of time characters identifying when a certain medical event occurred, or when the description of the medical event was recorded. Elements other than the timestamp and the user identifier may be treated as data elements as described above. Each data element may have at least one named attribute definition, which defines the type of data element.

For each input data model, there may be at least one timestamp, one patient identifier and one data element. The information may be encoded or maintained in free text, in which case text analysis tools may be employed to parse the free text.

For each data element, the query generated from the input data model may be used to automatically extract the relevant data. The data elements in the digital report form may be mapped to different driver events. A driver event may include a specific disease treatment operation method, such as Transhepatic Arterial Chem Otherapy And Embolization (TACE), liver transplantation, hepatectomy, alinjection that are relevant to the treatment of oncology. As the user data is extracted based on the driver events and multiple driver events may appear in one digital report form, the extracted user data may need to be merged accordingly.

In other words, the invention provides a method of parsing a digital report form, or CRF, into a list of questions to be answered using user data, thereby filling the report form automatically. Put another way, each data field defined in a CRF may be transformed into a question and, for each question, elements such as: a timestamp; a semantic definition; a document type; and any other context information, such as units for lab results, may be used to answer the question.

The method may be employed as part of a module that independently develops new, or integrates existing, phenotyping algorithms for filling digital report forms.

In addition to filling the digital report form automatically using the relevant user data extracted from the digital user record based on the query, the method may also include generating a data alert, such as an error flag or any other suitable indicator, for displaying to a user. For example, if no relevant user data can be extracted from the digital user record, the data alert may be displayed to the user in order to prompt the user into providing the missing relevant user data in order to completely fill the digital report form. In other words, the method may cause a processing system to remind a user, who may be a patient or a clinician, to complete a necessary examination or fill out a given document when relevant user data is not available to answer a query.

It should be noted that the determining of the input data model, the generation of the queries and the extraction of the relevant user data may be performed by machine learning algorithms.

A machine-learning algorithm is any self-training algorithm that processes input data in order to produce or predict output data. Here, the input data comprises digital report form, the input data model or the queries and the output data comprises the input data model, the queries or the extracted relevant user data, respectively.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example digital report forms. The training output data entries correspond to example extracted relevant user data.

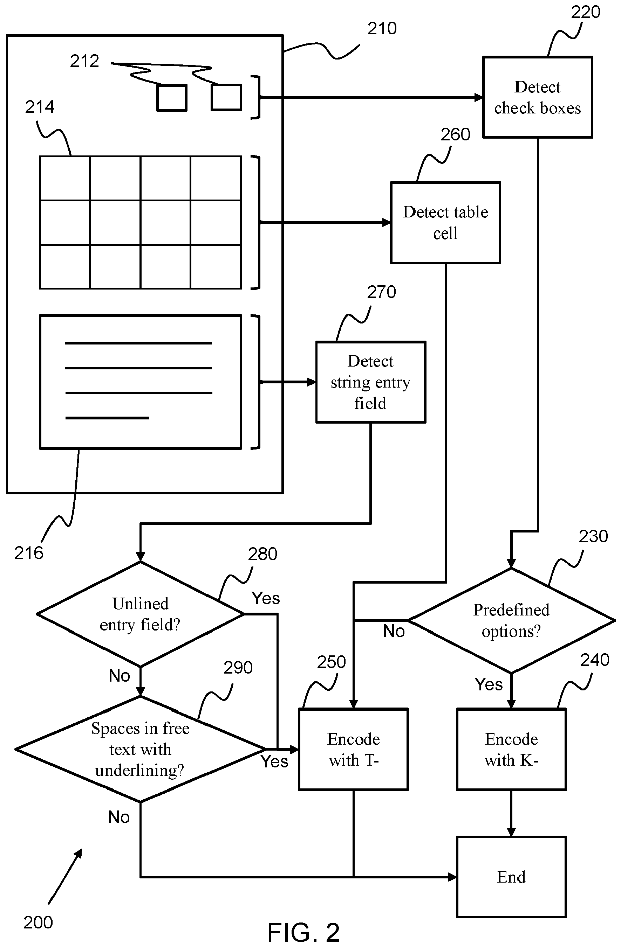

Figure 2 shows a schematic representation 200 of generating an ID for each data element from a data input model obtained from a digital report form 210, which comprises check boxes 212, a table 214 and a string entry field 216.

As an example, each data element may be divided into choice-style data element and free text type data element.

In step 220, the digital report form 210 is searched for check boxes 212 and in step 230 it is determined whether the check boxes have predefined options attached to them. If the check boxes do have predefined options attached to them, they are determined to be a choice-style query, and are encoded in step 240, for example, with a K identification, which may also include position information relating to the position of the check boxes on the digital report form. In a specific example, the check boxes may be encoded with identification code K-00003-3, the K indicating that the check boxes represent a choice-style input (such as a yes or no question), the 00003 indicating the number of the query (i.e. 00003 being the third choice-style data element of the digital report form), and the final number 3 indicating the length of original blank line for receiving the input data.

If in step 230 it is determined that the check boxes do not have predefined options associated with them, the check boxes may be considered as a free text-style data element and encoded in step 250, for example, with a T identification code.

In a specific example, the check boxes may be encoded with identification code T-00005-6, the T indicating that the check boxes represent a free text-style input (such date of operation), the 00005 indicating the number of the query (i.e. 00005 being the fifth free text type data element of the digital report form), and the final number 6 indicating the length of original blank line for receiving the input data.

In step 260, the digital report form 210 is searched for table cells 214, which may be treated as a free text type of data element and encoded with a T identification code.

In step 270, the digital report form 210 is searched for string entry fields 216. In step 280, it is determined whether there is an underlined entry field present in the digital report form. If there is an underlined entry field, the entry field is treated as a free text type of data element and encoded with a T identification code.

If there is no underlined entry field, it may be determined, in step 290, whether there are any spaces in the string entry field that have an underlined style applied. If there is a space with an underlined style applied, the space is treated as a free text type of data element and encoded with a T identification code.

If there is no underlined entry field or space with an underlined style applied, the string entry field may be identified as a fake data element and is not used to generate a query. This may apply, for example, for blocks of informational text on a digital report for that do not require any user data to be input. Where a fake data element is identified, a new data element may be identified within the digital report form. The new data element may then undergo the processes as described above and below.

By implementing the above-mentioned steps, each data element in the digital report form is extracted and is assigned a unique ID. Data elements determine the data input model.

When a medical professional designs an above-mentioned digital report from, some text based criterion are set up, so as to fill in patients' information or eligible user's information. Figure 3 shows a method 400 for identifying an eligible user for a clinical trial using a text based criterion, which may, for example, be found as part of a digital report form as described above. The step of obtaining 140 the digital user record of the method 100 is further based on the identified eligible user of the method 400 described in detail as below.

The assessment of the eligibility of a user for a clinical trial may be referred to as clinical phenotyping, wherein user data is used to detect their clinical phenotype according to one or more criteria. A user is eligible for a clinical trial if they possess the target clinical phenotype, which may be part of another, more complex clinical phenotype.

The method begins in step 410 by obtaining text data, wherein the text data comprises the text based criterion. The text data may be structured data and/or unstructured data and may be obtained by way of: natural language processing; a machine learning algorithm; and/or information extraction.

The text based criterion may include a temporal element. For clinical trials, a large proportion of the criteria may be temporally related criteria, for example, the start date of certain medication or the length of time a given symptom has been present. Further, the order of given medical events may be highly relevant to the clinical trial.

For example, a text based criterion may be as follows:
*Patients aged between 18 and 72 who received an electronic rofecoxib prescription and subsequently had a new code for myocardial infarction from the ICD-9 within five years.*

In step 420, the text based criterion is decomposed into one or more sub-sentences. For example, the above text based criterion may be decomposed as follows:
*Patients who received an electronic rofecoxib prescription and subsequently had a new code for myocardial infarction from the ICD-9 within five years.*
*Patients aged between 18 and 72.*

The relationship between the sub-sentences may be "all of', "any of' or "most of'. The sub-sentences may be assigned to a group, wherein the group comprises a general group and a first order difference group, wherein the first order difference group is dependent on the general group. The difference between the general group and the first order difference group is the relationship between sub-sentences. For the general group, the relationship may be all of, any of and most of. For the first order difference group, only "all of' relationships are permitted. Assigning of the sub-sentences to a group may be based on the temporal element.

The one or more sub-sentences may be provided to a user in order to receive a user input on the one or more sub-sentences. For example, the user may approve a sub-sentence or provide an alteration to a sub-sentence. The sub-sentences may then be updated based on the user input.

In step 430, the one or more sub-sentences are decomposed into one or more semantic phrases. For example, the above sub-sentences may be decomposed as follows:
*Patients who received an electronic rofecoxib prescription.*
*Patients who subsequently had a new code for myocardial infarction from the ICD-9 within five years.*
*Age > 18*
*Age < 72*

The semantic phrases may also be grouped as described above. In the above example, the first semantic phrase would belong to the general group and the second semantic phrase would belong to the first order difference group.

In other words, the initial complex criterion is split into multiple phrases. Further, each phrase could also be further split into multiple phrases if required. This process may be performed manually by a user or by way of an NLP tool.

In step 440, each semantic phrase is identified as a search feature.

The search feature may comprise an entity of the text based criterion, wherein the entity comprises one or more of: a medicament identity, such as the name of a medicament; a medical condition; a laboratory; and a medical examination, such as a diagnostic test. Further, the search feature may comprise a feature of the text based criterion, wherein the feature of the text based criterion comprises one or more of: an arithmetic comparator; an affirmation; a negation; and a conditional statement. In addition, the search feature may comprise a value of the text based criterion, wherein the value of the text based criterion comprises one or more of: a numerical value; a numerical range; and a unit.

If the semantic phrase is a simple criterion, such as: a single clinical concept (for example, pregnant), a negation (for example, not pregnant), or a simple quantitative comparison (for example, white blood cell count (WBC) > 5000 cells/mm3), which may be detected by a concept value model, the sematic phrase may be split into entity, feature and value.

The entity may be semantically recognized as medication, laboratory and the like. Then, a corresponding user database resource may be mapped for data query based on the recognized entity, for example, by narrowing the search field to only users associated with a given medication. The feature may be semantically recognized as a negation, comparator and the like in order to once again narrow down the user database. The value may be used to compare with the remaining data in the user database. Finally, with logic operators, a logic tree is built and final result may be calculated.

In step 450, a search criterion is generated based on the one or more search features. The search feature may be compared to a medical database and updated based on the comparison.

In step 460, the user database is searched based on the search criterion and in step 470 an eligible user is identified based on the search of the user database.

As a patient may experience many events across the span of a given therapy regime, the method may account for a priority scale in the screening criteria for research subject selection. For a specific condition period, an index slot and time interval around the event can anchor the initial type of the event. Then, with the help of other qualifying conditions, a patient with special condition can be identified. The timestamps of different events and their relationship need to be parsed within the scope of clinical meaning in order to reduce the selection bias. According, timestamp information may be shared between different groups of selection criteria.

For example, some selection criteria have a required wash out period. In a specific example, a patient who took Warfarin, typically requires a 6 to 12 month wash out.

For a given user, there may be more than one Warfarin taken across their therapy, meaning each drug exposure needs to be checked to confirm whether the wash out period has been completed. The Warfarin taken event may be treated as an index point, and the wash out period is a secondary variable. The secondary variable can be calculated with the index event timestamp and any additional constraint conditions. Accordingly, the secondary variable will become an additional selection criterion for eligible user selection.

For a clinical trial, if there is condition with a secondary variable, such as a wash out period, in the timeline of a user, the user may be included as research subject. For a cohort study or case control study, if such a secondary condition is detected, the patient will enter the cohort or group, and the timestamp of the secondary variable will contribute to the cohort type.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (100) for automatically filling a digital report form with relevant user data, the method comprising:
obtaining (110) a digital report form;
extracting (120) an input data model from the digital report form;
generating (130) a query based on the input data model;
obtaining (140) a digital user record;
identifying (150) relevant user data in the digital user record based on the query;
extracting (160) the relevant user data; and
filling (170) the digital report form based on the relevant user data.

2. A method (100) as claimed in claims 1, wherein the method further comprises obtaining context information from the digital report form, and wherein the generating of a query is further based on the context information.

3. A method (100) as claimed in claim 2, wherein generating the query comprises:
grouping multiple entities of the context information from at least the digital report form;
generating multiple query sequences based on entities in at least one group; and
deriving the query of the input data model by comparing the multiple query sequences with the input data model.

4. A method (100) as claimed in claim 1, wherein the input data model comprises:
a timestamp;
a user identifier; and
a data element.

5. A method (100) as claimed in claim 4, wherein the data element comprises one or more of
a semantic definition;
a document type;
a numerical value;
a numerical range; and
a unit.

6. A method (100) as claimed in claim 5, wherein the semantic definition comprises one or more of:
a conditional statement;
a confirmation; and
a negation.

7. A method (100) as claimed in claim 1, wherein extracting the input data model comprises extracting a data element, and wherein extracting the data element comprises:
determining if the data element comprises:
a check box;
a table cell; or
a string entry field;
if the data element comprises a check box, determining if the check box comprises a predefined option;
if the check box comprises a predefined option, identifying the data element as a finite choice;
if the check box does not comprise a predefined option, identifying the data element as a free text entry;
if the data element comprises a table cell, identifying the data element as a free text entry; and
if the data element comprises a string entry field, identifying the data element as a free text entry.

8. A method (100) as claimed in claim 1, wherein the method further comprises:
identifying a data element of the input data model as a fake data element;
discarding the fake data element; and
obtaining a new data element from the digital report form.

9. A method (100) a claimed in claim 2, wherein the obtaining of the context information comprises applying a top-down algorithm, the top-down algorithm comprising:
identifying a page of the digital report form;
identifying a heading on the page; and
deriving the context information based on the heading.

10. A (100) method a claimed in claim 2, wherein the obtaining of the context information comprises applying a bottom-up algorithm, the bottom-up algorithm comprising:
applying a leaf entity matching algorithm to an entity of the digital report form;
identifying a similar entity based on the leaf matching of the entity; and
deriving the context information based on the similar entity.

11. A (100) method as claimed in claim 1, wherein the method further comprises generating a data alert for displaying to a user.

12. A method (100) as claimed in claims 1, wherein the method further comprises, if no relevant user data can be extracted, receiving a user input to provide relevant user data.

13. A method (100) as claimed in claims 1, wherein the method further comprises identifying (400) an eligible user using a text based criterion and obtaining (140) the digital user record is further based on the identified eligible user, wherein the method (400) of identifying the eligible user comprises:
obtaining (410) text data, wherein the text data comprises the text based criterion;
decomposing (420) the text based criterion into one or more sub-sentences;
decomposing (430) the one or more sub-sentences into one or more semantic phrases;
identifying (440) each semantic phrase as a search feature;
generating (450) a search criterion based on the one or more search features;
searching (460) a user database based on the search criterion; and
identifying (470) an eligible user based on the search of the user database.

14. A method (100) as claimed in claim 13, wherein the text based criterion comprises a temporal element and wherein the search feature comprises a temporal criterion.

15. A method (100) as claimed in claim 13, wherein the method further comprises assigning the sub-sentences to a group, wherein the group comprises:
a general group; and
a first order difference group, wherein the first order difference group is dependent on the general group.

16. A method (100) as claimed in claim 15, wherein the assigning of the sub-sentences to a group is based on a temporal element.

17. A method (100) as claimed in claim 13, wherein the method further comprises:
comparing the search feature to a medical database; and
updating the search criterion based on the comparison.

18. A method (100) as claimed in claim 13, wherein the method further comprises:
providing the one or more sub-sentences to a user;
receiving a user input on the one or more sub-sentences; and
updating the sub-sentences based on the user input.

19. A method (100) as claimed in claims 13, wherein the method further comprises generating a logic tree based on the search criterion.

20. A computer program comprising computer program code means which is adapted, when said computer program is run on a computer, to implement the method of claim 1.

21. A system for automatically filling a digital report form with relevant user data, the system comprising a processor adapted to:
obtain a digital report form;
extract an input data model from the digital report form;
generate a query based on the input data model;
obtain a digital user record;
identify relevant user data in the digital user record based on the query;
extract the relevant user data; and
fill the digital report form based on the relevant user data.
